# EUROPÄISCHE PATENTSCHRIFT

(11) **EP 0 694 537 B1**
(45) Veröffentlichungstag und Bekanntmachung des Hinweises auf die Patenterteilung: **19.09.2001**
(21) Anmeldenummer: 95108707.1
(22) Anmeldetag: 07.06.1995
(51) Int. Cl.: C07D 237/14, C07D 239/34, C07D 241/18, C07D 237/16, C07D 213/643, C07D 213/65, C07D 213/68, C07D 213/69, C07D 213/89, C07D 207/46, A61K 31/50, A61K 31/505, A61K 31/495, A61K 31/44, A61K 31/40

(54) **1,6-Dihydro-6-oxo-3-pyridazinyloxy-benzoylguanidin-Derivate, deren Herstellung und deren Verwendung als Inhibitoren des zellulären Na+/H+-Antiporters**
1,6-Dihydro-6-oxo-3-pyridazinyloxy-benzoylguanidine derivatives, their preparation and their use as inhibitors of the cellular Na+/H+-antiporter
Dérivés de 1,6-dihydro-6-oxo-3-pyridazinyloxy-benzoylguanidine, leur préparation et leur utilisation comme inhibiteurs de Na+/H+-antiporter cellulaire

(30) Priorität: 20.06.1994 DE 4421495
(43) Veröffentlichungstag der Anmeldung: 31.01.1996
(73) Patentinhaber: MERCK PATENT GmbH, 64293 Darmstadt (DE)
(72) Erfinder: Gericke, Rolf, Dr., D-64342 Seeheim-Jugenheim (DE); Dorsch, Dieter, Dr., D-64372 Ober-Ramstadt (DE); Baumgarth, Manfred, Dr., D-64297 Darmstadt (DE); Minck, Klaus-Otto, Dr., D-64372 Ober-Ramstadt (DE); Beier, Norbert, Dr., D-64354 Reinheim (DE)

(56) Entgegenhaltungen:
- EP-A- 0 416 499
- EP-A- 0 556 672
- EP-A- 0 589 336
- EP-A- 0 600 371
- EP-A- 0 602 523
- EP-A- 0 612 723

## Beschreibung

Die Erfindung betrifft Heterocyclyloxy-benzoylguanidine ausgewählt aus der Gruppe
a) N-Diaminomethylen-3-methylsulfonyl-4-(1,6-dihydro-6-oxo-3-pyridazinyloxy)-benzamid,
b) N-Diaminomethylen-3-methylsulfonyl-4-(1-methyl-1,6-dihydro-6-oxo-3-pyridazinyloxy)-benzamid,
c) N-Diaminomethylen-3-methylsulfonyl-4-(1,6-dihydro-6-oxo-3-pyridazinyloxy)-6-methyl-benzamid,
d) N-Diaminomethylen-3-methylsulfonyl-4-(1,6-dihydro-6-oxo-3-pyridazinyloxy)-6-chlor-benzamid,
e) N-Diaminomethylen-3-methylsulfonyl-4-(1,6-dihydro-6-oxo-3-pyridazinyloxy)-6-isopropyl-benzamid,
f) N-Diaminomethylen-3-methylsulfonyl-4-(1,6-dihydro-6-oxo-3-pyridazinyloxy)-6-trifluormethyl-benzamid,
g) N-Diaminomethylen-3-methylsulfonyl-4-(1-isopropyl-1,6-dihydro-6-oxo-3-pyridazinyloxy)-benzamid,
h) N-Diaminomethylen-3-methylsulfonyl-4-(1-propyl-1,6-dihydro-6-oxo-3-pyridazinyloxy)-benzamid,
i) N-Diaminomethylen-3-methylsulfonyl-4-(1-ethyl-1,6-dihydro-6-oxo-3-pyridazinyloxy)-benzamid,
sowie deren physiologisch unbedenklichen Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen.

Bei den neuen Verbindungen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 87, 378-384 (1992)) und die somit gute Antiarrhythmika darstellen, die sich insbesondere zur Behandlung von Arrhythmien eignen, die als Folge von Sauerstoffmangel auftreten.

Der bekannteste Wirkstoff der Gruppe der Acylguanidine ist Amilorid. Diese Substanz zeigt jedoch in erster Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.
Darüber hinaus kennt man strukturell ähnliche Verbindungen beispielsweise aus EP 04 16 499.

Die erfindungsgemäßen Substanzen der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so daß die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zellproliferation bedingten Erkrankungen wie Arteriosklerose, diabetische Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Substanzen zur diagnostischen Anwendung zur Erkennung von Krankheiten, die von einer gesteigerten Aktivität des Na⁺/H⁺-Antiporters z.B. in Erythrozyten, Thrombozyten oder Leukozyten begleitet werden.

Die Wirkungen der Verbindungen können mit Hilfe an sich bekannter Methoden ermittelt werden, wie sie z.B. von N. Escobales and J. Figueroa in J. Membrane Biol. 120, 41-49 (1991) oder von L. Counillon, W. Scholz, H.J. Lang und J. Pouysségur in Mol. Pharmacol. 44, 1041-1045 (1993) angegeben werden.
Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe Verwendung finden.

Gegenstand der Erfindung ist ferner ein Verfahren zur Herstellung von Verbindungen gemäß Anspruch 1 sowie von deren Salzen, dadurch gekennzeichnet, daß man Verbindungen ausgewählt aus der Gruppe
a) 3-Methylsulfonyl-4-(1,6-dihydro-6-oxo-3-pyridazinyloxy)- benzoesäure,
b) 3-Methylsulfonyl-4-(1-methyl-1,6-dihydro-6-oxo-3-pyridazinyloxy)-benzoesäure,
c) 3-Methylsulfonyl-4-(1,6-dihydro-6-oxo-3-pyridazinyloxy)-6-methylbenzoesäure,
d) 3-Methylsulfonyl-4-(1,6-dihydro-6-oxo-3-pyridazinyloxy)-6-chlorbenzoesäure,
e) 3-Methylsulfonyl-4-(1,6-dihydro-6-oxo-3-pyridazinyloxy)-6-isopropylbenzoesäure,
f) 3-Methylsulfonyl-4-(1,6-dihydro-6-oxo-3-pyridazinyloxy)-6-trifluormethylbenzoesäure,
g) 3-Methylsulfonyl-4-(1-isopropyl-1,6-dihydro-6-oxo-3-pyridazinyloxy)-benzoesäure,
h) 3-Methylsulfonyl-4-(1-propyl-1,6-dihydro-6-oxo-3-pyridazinyloxy)-benzoesäure,
i) 3-Methylsulfonyl-4-(1-ethyl-1,6-dihydro-6-oxo-3-pyridazinyloxy)-benzoesäure,
oder die entsprechenden Benzoylchlorid- oder Benzoesäuremethylester-Derivate mit Guanidin umsetzt,
und/oder
daß man eine Base einer erfindungsgemäßen Verbindung durch Behandeln mit einer Säure in eines ihrer Salze umwandelt.

Die Verbindungen werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den erfindungsgemäßen Verbindungen umsetzt.

Vorzugsweise werden die erfindungsgemäßen Verbindungen hergestellt, indem man ein aktiviertes Carbonsäurederivat, wobei die Carbonsäure-OH-Gruppe besonders bevorzugt Cl oder -O-CH₃ ist, mit Guanidin umsetzt. Besonders geeignet sind Reaktionsvarianten, bei denen die freie Carbonsäure in an sich bekannter Weise zu dem jeweiligen aktivierten Derivat umgesetzt und dieses dann direkt, ohne Zwischenisolierung, mit Guanidin zur Reaktion gebracht wird. Methoden, bei denen eine Zwischenisolierung entbehrlich ist, sind beispielsweise eine Aktivierung mit Carbonyldiimidazol, Dicyclohexylcarbodiimid oder die Mukayama-Variante (Angew. Chem. 91, 788-812 (1979).

Die Carbonsäuren werden durch nucleophile aromatische Substitution ausgehend von geeigneten Benzoesäurederivaten durch Umsetzung mit entsprechenden heterocyclischen Verbindungen hergestellt.

Besonders geeignete Verbindungen sind beispielsweise 2-, 3- oder 4-Hydroxypyridine, welche gegebenenfalls zusätzliche Substituenten besitzen können, ferner aber auch 2-Hydroxypyrazine, 2-, 4- oder 5-Hydroxypyrimidine oder 3- oder 4-Hydroxypyridazine. Insbesondere sind die Trimethylsilyloxy-Derivate der genannten Heterocyclen geeignete Reaktionspartner.

Die Umsetzung eines reaktionsfähigen Carbonsäurederivates mit Guanidin erfolgt in an sich bekannter Weise vorzugsweise in einem protischen oder aprotischen polaren oder unpolaren inerten organischen Lösungsmittel. Geeignete Lösungsmittel werden nachfolgend für die nukleophile aromatische Substitution genannt. Besonders bevorzugte Solventien sind jedoch Methanol, THF, Dimethoxyethan, Dioxan oder daraus herstellbare Gemische sowie Wasser. Als Reaktionstemperatur sind beispielsweise Temperaturen zwischen 20° und dem Siedepunkt des Lösungsmittels geeignet. Die Reaktionszeiten liegen zwischen 5 Min. und 12 Std.. Es ist zweckmäßig, bei der Reaktion einen Säurefänger einzusetzen. Hierzu eignen sich jegliche Arten von Basen, die die Reaktion selbst nicht stören. Besonders geeignet ist jedoch die Verwendung von anorganischen Basen wie Kaliumcarbonat oder von organischen Basen wie Triethylamin oder Pyridin oder aber ein Überschuß des Guanidins.

Eine Base einer erfindungsgemäßen Verbindung kann mit einer Säure in das zugehörige Säureadditionssalz übergeführt werden. Für diese Umsetzung kommen Säuren in Frage, die physiologische unbedenkliche Salze liefern. So können anorganische Säuren verwendet werden, z.B. Schwefelsäure. Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure. Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure.

Die erfindungsgemäßen Verbindungen und ihre physiologisch unbedenklichen Salze könne zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen) in eine geeignete Dosierungsform gebracht werden.

Gegenstand der Erfindung sind ferner Mittel, insbesondere pharmazeutische Zubereitungen, enthaltend mindestens eine erfindungsgemäße Verbindung und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden.

Insbesondere für die topische Anwendung kommen auch liposomale Zubereitungen in Betracht. Die angegebenen Zubereitungen können sterilisiert sein und/oder Hilfsstoffe wie Geleit-, Konservierungs-, Stabilisierungs- und/oder Netzmittel, Emulgatoren, Salze zur Beeinflussung des osmotisches Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die erfindungsgemäßen Verbindungen und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden, von Angina pectoris, Infarkten, Ischämien des Nervensystems wie z.B. Schlaganfall oder Hirnödeme, von Schockzuständen und zur Präventivbehandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankunken, Fibrosen sowie Organhypertrophien und -hyperplasien.
Dabei werden die erfindungsgemäßen Substanzen in der Regel in Analogie zu bekannten Antiarrhythmika, z.B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

### Beispiel 1

Man rührt eine Lösung von 540 mg 3-Methylsulfonyl-4-(1,6-dihydro-6-oxo-3-pyridazinyloxy)-benzoesäure [erhältlich durch Umsetzung von 3-Methylsulfonyl-4-chlor-benzoesäure mit 3-Trimethylsilyloxy-6-oxo-1,6-dihydropyridazin] und 300 mg Carbonyldiimidazol in 15 ml THF zwei Stunden bei Raumtemperatur und fügt diese anschließend zu 383 mg Guanidin. Man rührt weitere zwei Stunden. Nach üblicher Aufarbeitung erhält man das N-Diaminomethylen-3-methylsulfonyl-4-(1,6-dihydro-6-oxo-3-pyridazinyloxy)-benzamid, F. 268-270°.

Analog erhält man durch Umsetzung von Guanidin
mit 3-Methylsulfonyl-4-(1,6-dihydro-6-oxo-3-pyridazinyloxy)-6-methylbenzoesäure das N-Diaminomethylen-3-methylsulfonyl-4-(1,6-dihydro-6-oxo-3-pyridazinyloxy)-6-methyl-benzamid;
mit 3-Methylsulfonyl-4-(1,6-dihydro-6-oxo-3-pyridazinyloxy)-6-chlorbenzoesäure das N-Diaminomethylen-3-methylsulfony(-4-(1,6-dihydro-6-oxo-3-pyridazinyloxy)-6-chlor-benzamid;
mit 3-Methylsulfonyl-4-(1,6-dihydro-6-oxo-3-pyridazinyloxy)-6-isopropylbenzoesäure das N-Diaminomethylen-3-methylsulfonyl-4-(1,6-dihydro-6-oxo-3-pyridazinyloxy)-6-isopropyl-benzamid;
mit 3-Methylsulfonyl-4-(1,6-dihydro-6-oxo-3-pyridazinyloxy)-6-trifluormethyl-benzoesäure das N-Diaminomethylen-3-methylsulfonyl-4-(1,6-dihydro-6-oxo-3-pyridazinyloxy)-6-trifluormethyl-benzamid.

### Beispiel 2

Zu einer Lösung von 928 mg Guanidin in 15 ml Methanol werden 1,1 g 3-Methylsulfonyl-4-(2-pyridyloxy)-benzoesäuremethylester [erhältlich durch Umsetzung von 3-Methylsulfonyl-4-chlor-benzoesäure mit 2-Hydroxypyridin und anschließende Veresterung des Produkts mit Methyliodid/K₂CO₃ in Dimethylformamid (DMF)] hinzugefügt. Man rührt 45 Minuten bei 50° und erhält nach Entfernung des Lösungsmittels und üblicher Aufarbeitung das N-Diaminomethylen-3-methylsulfonyl-4-(2-pyridyloxy)-benzamid, woraus nach Behandlung mit verdünnter wäßriger HCI-Lösung und Gefriertrocknung das entsprechende Hydrochlorid, F. 247-250° erhalten wird.

Analog erhält man durch Umsetzung von Guanidin
mit 3-Methylsulfonyl-4-(1-methyl-6-oxo-1,6-dihydro-3-pyridazinyloxy)-benzoesäuremethylester das N-Diaminomethylen-3-methylsulfonyl-4-(1-methyl-6-oxo-1,6-dihydro-3-pyridazinyloxy)-benzamid, Hydrochlorid, F. > 270°; F. (Base) 235-237°;
mit 3-Methylsulfonyl-4-(1-isopropyl-6-oxo-1,6-dihydro-3-pyridazinyloxy)-benzoesäuremethylester das N-Diaminomethylen-3-methylsulfonyl-4-(1-isopropyl-6-oxo-1,6-dihydro-3-pyridazinyloxy)-benzamid, Hydrochlorid;
mit 3-Methylsulfonyl-4-(1-propyl-6-oxo-1,6-dihydro-3-pyridazinyloxy)-benzoesäuremethylester das N-Diaminomethylen-3-methylsulfonyl-4-(1-propyl-6-oxo-1 ,6-dihydro-3-pyridazinyloxy)-benzamid, Hydrochlorid;
mit 3-Methylsulfonyl-4-(1-ethyl-6-oxo-1,6-dihydro-3-pyridazinyloxybenzoesäuremethylester das N-Diaminomethylen-3-methylsulfonyl-4-(1-ethyl-6-oxo-1,6-dihydro-3-pyridazinyloxy)-benzamid, Hydrochlorid.

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann beispielsweise in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg erfindungsgemäßem Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg eines erfindungsgemäßen Wirkstoffs werden in üblicher Weise in Hartgelatinekapseln gefüllt, so daß jede Kapsel 20 mg des Wirkstoffs enthält.

### Beispiel H: Ampullen

Eine Lösung von 1 kg erfindungsgemäßem Wirkstoff in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
a) N-Diaminomethylen-3-methylsulfonyl-4-(1,6-dihydro-6-oxo-3-pyridazinyloxy)-benzamid,
b) N-Diaminomethylen-3-methylsulfonyl-4-(1-methyl-1,6-dihydro-6-oxo-3-pyridazinyloxy)-benzamid,
c) N-Diaminomethylen-3-methylsulfonyl-4-(1,6-dihydro-6-oxo-3-pyridazinyloxy)-6-methyl-benzamid,
d) N-Diaminomethylen-3-methylsulfonyl-4-(1,6-dihydro-6-oxo-3-pyridazinyloxy)-6-chlor-benzamid,
e) N-Diaminomethylen-3-methylsulfonyl-4-(1,6-dihydro-6-oxo-3-pyridazinyloxy)-6-isopropyl-benzamid,
f) N-Diaminomethylen-3-methylsulfonyl-4-(1,6-dihydro-6-oxo-3-pyridazinyloxy)-6-trifluormethyl-benzamid,
g) N-Diaminomethylen-3-methylsulfonyl-4-(1-isopropyl-1,6-dihydro-6-oxo-3-pyridazinyloxy)-benzamid,
h) N-Diaminomethylen-3-methylsulfonyl-4-(1-propyl-1,6-dihydro-6-oxo-3-pyridazinyloxy)-benzamid,
i) N-Diaminomethylen-3-methylsulfonyl-4-(1-ethyl-1,6-dihydro-6-oxo-3-pyridazinyloxy)-benzamid,
sowie deren physiologisch unbedenklichen Salze.

2. Verfahren zur Herstellung von Verbindungen gemäß Anspruch 1 sowie von deren Salzen, **dadurch gekennzeichnet, daß** man Verbindungen ausgewählt aus der Gruppe
a) 3-Methylsulfonyl-4-(1,6-dihydro-6-oxo-3-pyridazinyloxy)-benzoesäure,
b) 3-Methylsulfonyl-4-(1-methyl-1,6-dihydro-6-oxo-3-pyridazinyloxy)-benzoesäure,
c) 3-Methylsulfonyl-4-(1,6-dihydro-6-oxo-3-pyridazinyloxy)-6-methylbenzoesäure,
d) 3-Methylsulfonyl-4-(1,6-dihydro-6-oxo-3-pyridazinyloxy)-6-chlorbenzoesäure,
e) 3-Methylsulfonyl-4-(1,6-dihydro-6-oxo-3-pyridazinyloxy)-6-isopropyl- benzoesäure,
f) 3-Methylsulfonyl-4-(1,6-dihydro-6-oxo-3-pyridazinyloxy)-6-trifluormethyl-benzoesäure,
g) 3-Methylsulfonyl-4-(1-isopropyl-1,6-dihydro-6-oxo-3-pyridazinyloxy)- benzoesäure,
h) 3-Methylsulfonyl-4-(1-propyl-1,6-dihydro-6-oxo-3-pyridazinyloxy)-benzoesäure,
i) 3-Methylsulfonyl-4-(1-ethyl-1,6-dihydro-6-oxo-3-pyridazinyloxy)-benzoesäure,
oder die entsprechenden Benzoylchlorid- oder Benzoesäuremethylester-Derivate
mit Guanidin umsetzt,
und/oder
daß man eine Base einer erfindungsgemäßen Verbindung durch Behandeln mit einer Säure in eines ihrer Salze umwandelt.

3. Verfahren zur Herstellung pharmazeutischer Zubereitungen, **dadurch gekennzeichnet, daß** man eine Verbindung ausgewählt aus den Verbindungen nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

4. Pharmazeutische Zubereitung, **gekennzeichnet durch** einen Gehalt an mindestens einer Verbindung ausgewählt aus den Verbindungen nach Anspruch 1 und/oder einem ihrer physiologisch unbedenklichen Salze.

5. Verwendung der Verbindungen nach Anspruch 1 und/oder einem ihrer physiologisch unbedenklichen Salze zur Herstellung eines Arzneimittels.

6. Verwendung der Verbindungen nach Anspruch 1 und/oder einem ihrer physiologisch unbedenklichen Salze zur Herstellung eines Arzneimittels zur Behandlung von Arrhythmien, Angina pectoris, Infarkten sowie zur präventiven Behandlung der genannten Indikationen.

## Claims

1. Compounds selected from the group
a) N-diaminomethylene-3-methylsulphonyl-4-(1,6-dihydro-6-oxo-3-pyridazinyloxy)benzamide,
b) N-diaminomethylene-3-methylsulphonyl-4-(1-methyl-1,6-dihydro-6-oxo-3-pyridazinyloxy)benzamide,
c) N-diaminomethylene-3-methylsulphonyl-4-(1,6-dihydro-6-oxo-3-pyridazinyloxy)-6-methylbenzamide,
d) N-diaminomethylene-3-methylsulphonyl-4-(1,6-dihydro-6-oxo-3-pyridazinlyoxy)-6-chlorobenzamide,
e) N-diaminomethylene-3-methylsulphonyl-4-(1,6-dihydro-6-oxo-3-pyridazinyloxy)-6-isopropylbenzamide,
f) N-diaminomethylene-3-methylsulphonyl-4-(1,6-dihydro-6-oxo-3-pyridazinyloxy)-6-trifluoromethylbenzamide,
g) N-diaminomethylene-3-methylsulphonyl-4- (1-isopropyl-1,6-dihydro-6-oxo-3-pyridazinyloxy)benzamide,
h) N-diaminomethylene-3-methylsulphonyl-4-(1-propyl-1,6-dihydro-6-oxo-3-pyridazinyloxy)benzamide,
i) N-diaminomethylene-3-methylsulphonyl-4-(1-ethyl-1,6-dihydro-6-oxo-3-pyridazinyloxy)benzamide,
and the physiologically harmless salts thereof.

2. Process for preparing compounds according to Claim 1, and their salts, **characterized in that** compounds selected from the group
a) 3-methylsulphonyl-4-(1,6-dihydro-6-oxo-3-pyridazinyloxy)benzoic acid,
b) 3-methylsulphonyl-4-(1-methyl-1,6-dihydro-6-oxo-3-pyridazinyloxy)benzoic acid,
c) 3-methylsulphonyl-4-(1,6-dihydro-6-oxo-3-pyridazinyloxy)-6-methylbenzoic acid,
d) 3-methylsulphonyl-4-(1,6-dihydro-6-oxo-3-pyridazinyloxy)-6-chlorobenzoic acid,
e) 3-methylsulphonyl-4-(1,6-dihydro-6-oxo-3-pyridazinyloxy)-6-isopropylbenzoic acid,
f) 3-methylsulphonyl-4-(1,6-dihydro-6-oxo-3-pyridazinyloxy)-6-trifluoromethylbenzoic acid,
g) 3-methylsulphonyl-4-(1-isopropyl-1,6-dihydro-6-oxo-3-pyridazinyloxy)benzoic acid,
h) 3-methylsulphonyl-4-(1-propyl-1,6-dihydro-6-oxo-3-pyridazinyloxy)benzoic acid,
i) 3-methylsulphonyl-4-(1-ethyl-1,6-dihydro-6-oxo-3-pyridazinyloxy)benzoic acid,
or the corresponding benzoyl chloride derivatives or methyl benzoate derivatives
are reacted with guanidine,
and/or
in that a base of a compound according to the invention is converted into one of its salts by being treated with an acid.

3. Process for producing pharmaceutical preparations, **characterized in that** a compound selected from amongst the compounds according to Claim 1 and/or one of its physiologically harmless salts is/are brought, together with at least one solid, liquid or semiliquid carrier substance or auxiliary substance, into a suitable dosage form.

4. Pharmaceutical preparation, **characterized by** a content of at least one compound selected from amongst the compounds according to Claim 1 and/or one of its physiologically harmless salts.

5. Use of the compounds according to Claim 1, and/or of a physiologically harmless salt thereof, for producing a medicament.

6. Use of the compounds according to Claim 1, and/or of a physiologically harmless salt thereof, for producing a medicament for the treatment of arrhythmias, angina pectoris and infarctions, and also for the preventive treatment of the said indications.

## Revendications

1. Composés choisis parmi le groupe constitué par
a) le N-diaminométhylène-3-méthylsulfonyl-4-(1,6-dihydro-6-oxo-3-pyridazinyloxy)-benzamide,
b) le N-diaminométhylène-3-méthylsulfonyl-4-(1-méthyl-1,6-dihydro-6-oxo-3-pyridazinyloxy)-benzamide,
c) le N-diaminométhylène-3-méthylsulfonyl-4-(1,6-dihydro-6-oxo-3-pyridazinyloxy)-6-méthyl-benzamide,
d) le N-diaminométhylène-3-méthylsulfonyl-4-(1,6-dihydro-6-oxo-3-pyridazinyloxy)-6-chloro-benzamide,
e) le N-diaminométhylène-3-méthylsulfonyl-4-(1,6-dihydro-6-oxo-3-pyridazinyloxy)-6-isopropyl-benzamide,
f) le N-diaminométhylène-3-méthylsulfonyl-4-(1,6-dihydro-6-oxo-3-pyridazinyloxy)-6-trifluorométhyl-benzamide,
g) le N-diaminométhylène-3-méthylsulfonyl-4-(1-isopropyl-1,6-dihydro-6-oxo-3-pyridazinyloxy)-benzamide,
h) le N-diaminométhylène-3-méthylsulfonyl-4-(1-propyl-1,6-dihydro-6-oxo-3-pyridazinyloxy)-benzamide,
i) le N-diaminométhylène-3-méthylsulfonyl-4-(1-éthyl-1,6-dihydro-6-oxo-3-pyridazinyloxy)-benzamide,
ainsi que leurs sels physiologiquement compatibles.

2. Procédé de production de composés selon la revendication 1, ainsi que leurs sels, **caractérisé en ce que** l'on transforme les composés choisis parmi le groupe constitué par
a) l'acide 3-méthylsulfonyl-4-(1,6-dihydro-6-oxo-3-pyridazinyloxy)-benzoïque
b) l'acide 3-méthylsulfonyl-4-(1-méthyl-1,6-dihydro-6-oxo-3-pyridazinyloxy)-benzoïque,
c) l'acide 3-méthylsulfonyl-4-(1,6-dihydro-6-oxo-3-pyridazinyloxy)-6-méthylbenzoïque,
d) l'acide 3-méthylsulfonyl-4-(1,6-dihydro-6-oxo-3-pyridazinyloxy)-6-chlorobenzoïque,
e) l'acide 3-méthylsulfonyl-4-(1,6-dihydro-6-oxo-3-pyridazinyloxy)-6-isopropyl- . benzoïque,
f) l'acide 3-méthylsulfonyl-4-(1,6-dihydro-6-oxo-3-pyridazinyloxy)-6-trifluorométhyl-benzoïque,
g) l'acide 3-méthylsulfonyl-4-(1-isopropyl-1,6-dihydro-6-oxo-3-pyridazinyloxy)-benzoïque,
h) l'acide 3-méthylsulfonyl-4-(1-propyl-1,6-dihydro-6-oxo-3-pyridazinyloxy)-benzoïque,
i) l'acide 3-méthylsulfonyl-4-(1-éthyl-1,6-dihydro-6-oxo-3-pyridazinyloxy)-benzoïque, ou les dérivés chlorure de benzoyle ou esters méthyliques de l'acide benzoïque correspondants
avec la guanidine
et/ou
que l'on transforme une base d'un composé selon la présente invention en son sel par traitement avec un acide.

3. Procédé de production de préparations pharmaceutiques, **caractérisé en ce que** l'on amène en une formulation appropriée un composé choisi parmi les composés selon la revendication 1 et/ou un de leurs sels physiologiquement compatibles, avec au moins un agent porteur ou un excipient solide, liquide ou semi-liquide.

4. Préparation pharmaceutique, **caractérisée par** une teneur en au moins un composé choisi parmi les composés selon la revendication 1 et/ou un de leurs sels physiologiquement compatibles.

5. Utilisation des composés selon la revendication 1 et/ou d'un de leurs sels physiologiquement compatibles pour l'élaboration d'un médicament.

6. Utilisation des composés selon la revendication 1 et/ou d'un de leurs sels physiologiquement compatibles, pour l'élaboration d'un médicament pour le traitement des arythmies, de l'angine de poitrine, de l'infarctus ainsi que pour le traitement préventif des indications susnommées.
